# EUROPEAN PATENT SPECIFICATION

(11) **EP 4 445 896 B1**
(45) Date of publication and mention of the grant of the patent: **08.04.2026**
(21) Application number: 21967320.9
(22) Date of filing: 08.12.2021
(51) Int. Cl.: A61K 9/10, A61K 9/14, A61K 9/107, A61K 47/38, A61K 47/22, A61K 47/12, A61K 31/37

(54) **NANOSUSPENSION CONTAINING PROGERININ, AND PREPARATION METHOD THEREFOR**
NANOSUSPENSION MIT PROGERININ UND HERSTELLUNGSVERFAHREN DAFÜR
NANOSUSPENSION CONTENANT DE LA PROGÉRINE ET SON PROCÉDÉ DE PRÉPARATION

(43) Date of publication of application: 16.10.2024
(73) Proprietor: PRG S&Tech Inc., Busan 46274 (KR)
(72) Inventor: KIM, Min Ju, Gijang-gun Busan 46081 (KR)
(74) Representative: Mewburn Ellis LLP
(86) International application number: PCT/KR2021/018550
(87) International publication number: WO 2023/106447

(56) References cited:
- WO-A1-2018/199633
- WO-A1-2018/199633
- KR-A- 20050 026 705
- KR-A- 20100 126 445
- US-A1- 2020 048 274
- GEORGE MAYA ET AL: "Identifying the correlation between drug/stabilizer properties and critical quality attributes (CQAs) of nanosuspension formulation prepared by wet media milling technology", EUROPEAN JOURNAL OF PHARMACEUTICAL SCIENCES- AUTHOR MANUSCRIPT, ELSEVIER AMSTERDAM, NL, vol. 48, no. 1, 22 October 2012 (2012-10-22), pages 142 - 152, XP028975341, ISSN: 0928-0987, DOI: 10.1016/J.EJPS.2012.10.004
- GEORGE MAYA; GHOSH INDRAJIT: "Identifying the correlation between drug/stabilizer properties and critical quality attributes (CQAs) of nanosuspension formulation prepared by wet media milling technology", EUROPEAN JOURNAL OF PHARMACEUTICAL SCIENCES, ELSEVIER AMSTERDAM, NL, vol. 48, no. 1, 22 October 2012 (2012-10-22), NL , pages 142 - 152, XP028975341, ISSN: 0928-0987, DOI: 10.1016/j.ejps.2012.10.004
- INDRAJIT GHOSH, SONALI BOSE, RADHA VIPPAGUNTA, FERRIS HARMON: "Nanosuspension for improving the bioavailability of a poorly soluble drug and screening of stabilizing agents to inhibit crystal growth", INTERNATIONAL JOURNAL OF PHARMACEUTICS, ELSEVIER, vol. 409, no. 1-2, 1 May 2011 (2011-05-01), pages 260 - 268, XP055112634, ISSN: 03785173, DOI: 10.1016/j.ijpharm.2011.02.051

## Description

### Technical Field

The present disclosure relates to a nanosuspension including progerinin which is a poorly soluble drug, and a preparation method thereof.

### Background Art

Many useful drugs are hydrophobic with low solubility in an aqueous medium, making it difficult to formulate as a suspension in an aqueous vehicle. In particular, due to this property, wetting agents are often required to promote suspension of hydrophobic drug particles in aqueous media. It is known that surface-active wetting agents (i.e., surfactants) such as sodium lauryl sulfate reduce the interfacial tension between the drug particles and the suspension vehicle to allow suspension vehicles to penetrate into drug aggregates and/or pores on the drug particle, thereby increasing suspension properties of hydrophobic drugs in the aqueous medium, at least partially. However, in addition to the favorable drug-suspension effect, the use of surfactants in the suspension also leads to undesirable consequences of the solubilized and/or dissolved free drugs.

Since solubilized and/or dissolved drugs are susceptible to chemical degradation and/or interactions with other components, suspensions containing free drugs may be chemically unstable. Another undesirable consequence of using relatively high amounts of surfactant to promote suspension of low soluble drugs is that because surfactants stabilize bubbles, accompanying air tends to remain trapped during homogenization or shaking of these suspensions. Since this trapped air changes with the stirring force, duration of the stirring, and an elapsed time of the stirring, the volume of the suspension changes, making it difficult or impossible to administer at a uniform dose over time.

If a low water-soluble drug is administered as a suspension, it is desirable that the suspension shows gentle sedimentation to provide adequate uniformity in the dose. Conversely, if rapid sedimentation occurs, as the case of the vehicle, the suspension must be shaken before each administration in order to achieve uniformity in the dose. If other factors (such as drug particle size, uniformity, and density) are equivalent, a sinking rate of drug particles decreases as a viscosity of a particular suspension vehicle increases. Therefore, it is desirable that the suspension be moderately viscous to inhibit or slow down the sedimentation of drug particles. However, while this increased viscosity promotes physical stability, it also makes it difficult to pour or administer the suspension.

On the other hand, progerinin, which has a structure represented by the following Chemical Formula 1, has shown excellent effects of suppressing progerin expression and effects of inhibiting binding between progerin and lamin A, and, as a drug exhibiting effects of prolonging a survival period of progeria-induced animal model, it may be used as a pharmaceutical composition for preventing or treating progeria.

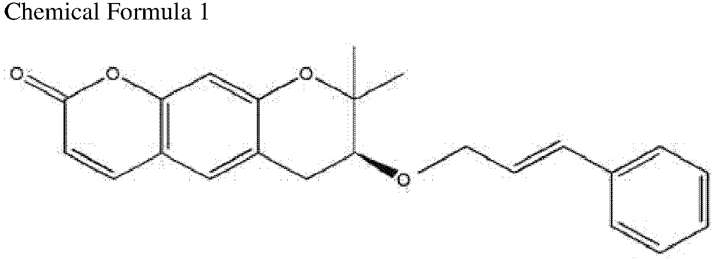

The compound name of the progerinin is (7S)-(+)-8,8-dimethyl-7-(3-phenylallyloxy)-7,8-dihydro-6H-pyrano [3,2-g]chromen-2-one (named 'SLC-D011').

Progerinin, a drug that has been shown to be effective in progeria, is a BCS class II molecule exhibiting high apparent permeability and low solubility in aqueous media according to the criteria of the Biopharmaceutical Classification System (BCS).

Due to the low water solubility of progerinin drugs, their therapeutic applications are very limited. To overcome these limitations, a number of methods have been studied, such as the use of polymeric nanoparticles, solid lipid nanoparticles, autologous emulsification drug delivery systems, nanoemulsions, liposomes, nanosuspensions, and nanofibers. Thereamong, the nanosuspension is a colloidal dispersion of stabilized drug particles in the presence of polymers, surfactants, or both. The nanosuspension is used to deliver drug substances that exhibit low water solubility and lipid solubility. The small particles in the nanosuspension provide a very large drug surface area, thereby increasing a dissolution rate of insoluble drugs. Consequently, BCS class II and IV compounds exhibit enhanced bioavailability, rapid activity, and other desirable biopharmaceutical effects.

Under these circumstances, the inventors were able to conduct animal experiments using a mixture of monoolein and tricaprin, which are oil-based solutions, but due to the nature of the disease, it was found that an oil-dissolved formulation is incompatible as high doses and long-term intake are required, recognizing a need for development of a new formulation that allows a long-term intake. In particular, the above-mentioned progerinin is poorly soluble to the extent that its solubility in water is close to zero, and the solubility is very low even in existing ingestible solvents, such that it is necessary to develop a formulation that enables long-term intake and body absorption through the introduction of new technology.

Therefore, the inventors have attempted to develop a nanosuspension formulation that is suitable for progerinin drugs with effects of preventing or treating progeria, and a preparation method thereof. Specifically, by optimizing the particle diameter of progerinin drugs with excellent bioabsorption efficiency and selecting water-soluble polymers and excipients for preparation of nanosuspensions, it was aimed to develop an oral progerinin nanosuspension preparation including progerinin drugs with outstanding dispersibility or uniformity and enhanced bioavailability as well as enhanced stability.

### Disclosure

### Technical Goals

An object of the present disclosure is to provide a method of preparing a nanosuspension including a progerinin drug with excellent bioavailability. Another object of the present disclosure is to provide a formulation with enhanced bioavailability of a drug and stability while ensuring easiness in oral intake.

### Technical Solutions

To achieve the above objects, the present disclosure provides a nanosuspension including progerinin, including (a) 1 to 10 wt% of a progerinin compound represented by the following Chemical Formula 1; (b) 0.5 to 5 wt% of hydroxypropyl methylcellulose (HPMC) which is a polymeric suspension; (c) 0.5 to 5 wt% of D-α-tocopherol polyethylene glycol succinate (TPGS) which is a solubility enhancer; and (d) 0.1 to 0.5 wt% of potassium sorbate which is a preservative, wherein the progerinin compound is drug particles formed via wet type ball milling in an average particle diameter (D50) of 100 nm to 300 nm.

To achieve another object above, the present disclosure provides a nanosuspension including progerinin, including (a) 1 to 10 wt% of a progerinin compound represented by the following Chemical Formula 1; (b') 0.5 to 5 wt% of hydroxypropyl cellulose (HPC) which is a polymeric suspension; (c') 0.1 to 0.5 wt% of dioctyl sodium sulfosuccinate (DOSS) which is a surfactant; and (d) 0.1 to 0.5 wt% of potassium sorbate which is a preservative, wherein the progerinin compound is drug particles formed via wet type ball milling in an average particle diameter (D50) of 100 nm to 300 nm.

To achieve the above objects, the present disclosure provides a method of preparing a nanosuspension including progerinin, including (i) mixing purified water with hydroxypropyl methylcellulose (HPMC) (b) and D-α-tocopherol polyethylene glycol succinate (TPGS) (c) to prepare a vehicle solution and then adding and mixing progerinin (a) represented by the following Chemical Formula 1 so as to prepare a suspension; (ii) subjecting the suspension to wet type ball milling to prepare a nanosuspension; and (iii) mixing the nanosuspension with potassium sorbate (d) to finally prepare a nanosuspension with enhanced stability, wherein the progerinin compound in the nanosuspension of (iii) is in the form of drug particles with an average particle diameter (D50) of 100 nm to 300 nm.

To achieve another object above, the present disclosure provides a method of preparing a nanosuspension including progerinin, including (i) mixing purified water with hydroxypropyl cellulose (HPC) (b'); and dioctyl sodium sulfosuccinate (DOSS) (c'); to prepare a vehicle solution and then adding and mixing progerinin (a) represented by the following Chemical Formula 1 so as to prepare a suspension; (ii) subjecting the suspension to wet type ball milling to prepare a nanosuspension; and (iii) mixing the nanosuspension with potassium sorbate (d) to finally prepare a nanosuspension with enhanced stability, wherein the progerinin compound in the nanosuspension of (iii) is in the form of drug particles with an average particle diameter (D50) of 100 nm to 300 nm.

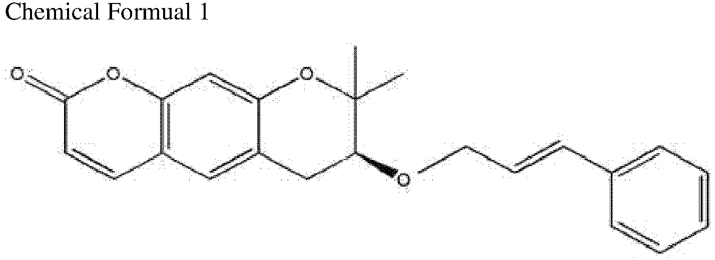

### Advantageous Effects

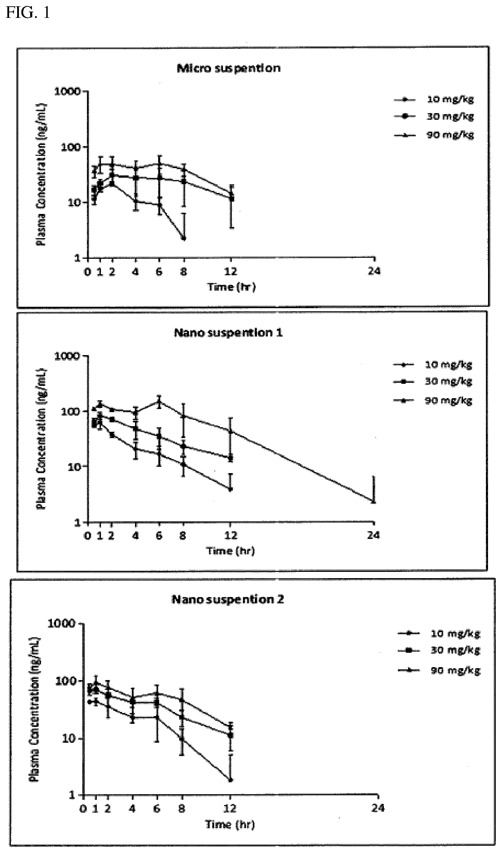

The present disclosure relates to a nanosuspension formulation suitable for a poorly soluble progerinin drug and a preparation method thereof, wherein, in the case of the nanosuspension according to the present disclosure which includes drug particles with an average particle diameter of 200 nm or less and is obtained by mixing micronized progerinin drugs with hydroxypropyl methylcellulose (HPMC) or hydroxypropyl cellulose (HPC), a water-soluble polymer; D-α-tocopherol polyethylene glycol succinic acid (TPGS), a solubility agent, or dioctyl sodium sulfosuccinate (DOSS), a surfactant; and potassium sorbate, a preservative; and then subjecting the mixture to ball milling in a dyno-mill chamber for a predetermined period of time, excellent bioavailability was exhibited (Example 1-1 and FIG. 1).

As such, while the nanosuspension including the progerinin according to the present disclosure as an active ingredient has uniform dispersibility and stability, as a result of the experiment, it was determined that the nanosuspension has dispersion stability and excellent bioabsorption efficiency by maintaining particle homogeneity of 200 nm or less for more than 20 days (up to 70 days) at 4°C.

### Brief Description of Drawings

FIG. 1 shows results of pharmacokinetic analysis of a progerinin suspension.
FIG. 2 shows a result of measuring a solubility of a solid dispersion prepared via hot melt extrusion (HMT).
FIG. 3 shows a result of measuring a solubility of a solid dispersion prepared via a hot melt extrusion process with polymers in various ratios (1:1).
FIG. 4 shows a result of pharmacokinetic analysis on a solid dispersion prepared via a hot melt extrusion process.
FIG. 5 shows results of measuring a solubility of an amorphous solid dispersion via a spray drying process (A: pH 1.2, B: pH 6.8).
FIG. 6 shows a result of pharmacokinetic analysis on a solid dispersion prepared via a spray drying process.
FIG. 7 shows images of a nanosuspension prepared via wet type ball milling with beads (top) and a result of measuring an average particle diameter of a progerinin drug (bottom).
FIG. 8 shows a process of preparing a nanosuspension according to the present disclosure.

### Best Mode for Carrying Out the Invention

Hereinafter, the present disclosure will be described in more detail.

The inventors have completed a method of preparing a progerinin nanosuspension with enhanced bioavailability, wherein, in the case of the nanosuspension that is obtained by mixing micronized progerinin drugs in a vehicle solution including hydroxypropyl methylcellulose (HPMC), D-α-tocopherol polyethylene glycol succinate (TPGS), and potassium sorbate and then subjecting the mixture to wet type ball milling in a dyno-mill chamber for a predetermined period of time, it has uniform particle dispersibility by including particles with an average particle diameter (D50) of 200 nm or less and also ensures stability as the dispersibility is maintained for more than 20 days at 4°C.

Thus, the present disclosure provides a nanosuspension including progerinin with enhanced bioavailability.

Specifically, as an example, a nanosuspension according to the present disclosure incudes (a) 1 to 10 wt% of a progerinin compound represented by the following Chemical Formula 1; (b) 0.5 to 5 wt% of hydroxypropyl methylcellulose (HPMC) which is a polymeric suspension; (c) 0.5 to 5 wt% of D-α-tocopherol polyethylene glycol succinate (TPGS) which is a solubility enhancer; and (d) 0.1 to 0.5 wt% of potassium sorbate which is a preservative.

As another example embodiment, a nanosuspension according to the present disclosure includes (a) 1 to 10 wt% of a progerinin compound represented by the following Chemical Formula 1; (b') 0.5 to 5 wt% of hydroxypropyl cellulose (HPC) which is a polymeric suspension; (c') 0.1 to 0.5 wt% of dioctyl sodium sulfosuccinate (DOSS) which is a surfactant; and (d) 0.1 to 0.5 wt% of potassium sorbate which is a preservative.

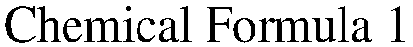

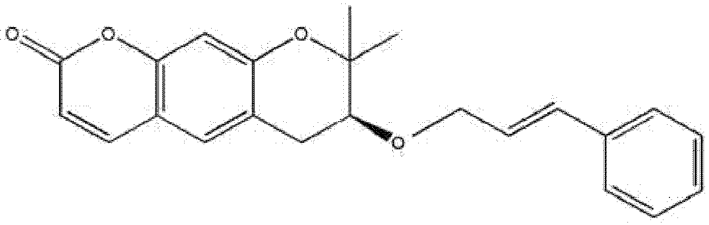

The progerinin (a) is a decursin derivative which is (7S)-(+)-8,8-dimethyl-7-(3-phenylallyloxy)-7,8-dihydro-6H-pyrano[3,2-g]chromen-2-one. In the present disclosure, progerinin may also be named 'SLC-D011'.

Progerinin drugs have shown excellent effects of suppressing progerin expression and effects of inhibiting binding between progerin and lamin A and may be used as a pharmaceutical composition for preventing or treating progeria, an age-related disease, as a drug that has an effect of prolonging a survival period in progeria-induced animal models. While progerinin is poorly soluble that its solubility in water is close to zero, in the present disclosure, solubility is enhanced, and a formulation that enables long-term intake and absorption in the human body is provided.

Specifically, hydroxypropyl methylcellulose (HPMC) (b) or hydroxypropyl cellulose (HPC) (b'), which corresponds to a water-soluble polymer that is a polymeric suspension agent in the present disclosure, is an ingredient that helps dispersion of progerinin drugs and other components in a solution. It is desirable that the polymeric suspension agent is included in an amount of 0.5 to 5 wt% with respect to the total nanosuspension. More preferably, the amount is 1 to 3 wt%.

If the polymeric suspension agent is included below the range, the sinking rate of the suspension becomes poor, and if the polymeric suspension agent is included above the range, preparation becomes challenging due to difficulty in stirring. In particular, if the polymeric suspension agent is included above the range, the dispersibility of the suspension is lowered due to poor uniformity in the stirring, thereby resulting in an increase in the volume of defective products with insufficiency in the content in the production of the product.

In the present disclosure, D-α-tocopherol polyethylene glycol succinate (TPGS) (c), a solubility enhancer, is a component that enhances the solubility of a poorly soluble drug. It is desirable that the TPGS is included in an amount of 0.5 to 5 wt% with respect to the total nanosuspension. More preferably, the amount is 1 wt%. If the solubility enhancer is included below the range, the solubility is lowered, and if the solubility enhancer is included above the range, the toxicity permissible limit may be exceeded, such that it is recommended to use it within the above range.

In addition, in the present disclosure, dioctyl succinate sodium (DOSS) (c'), a surfactant, may be used instead of the solubility enhancer. Here, it is desirable to use the surfactant in an amount of 0.1 to 0.5 wt%. More preferably, the amount is 0.25wt%. If the surfactant is included below the range, the solubility becomes lowered, and if the surfactant is included above the range, the toxicity permissible limit may be exceeded, such that it is recommended to use it within the above range.

It is desirable that the progerinin drug, which is a poorly soluble drug in the suspension, has an average particle diameter (D50) of 100 nm to 300 nm. More preferably, the average particle diameter is 150 nm to 250 nm, and even more preferably, the average particle diameter is 200 nm or less. With the average particle diameter within the above range, the solubility is the highest, and nanoparticles have a larger surface area to be dissolved more easily, thereby maximizing the bioavailability or bioabsorption efficiency. This was determined by Example <1-1> that is described below. Moreover, for the preparation of nanosuspensions having the average particle diameter, it may be prepared via wet type ball milling using a dyno-mill.

As used herein, the term "stability" may all include homogeneity, dispersibility, sedimentation stability, and storage stability of drug particles. In particular, the nanosuspension according to the present disclosure has uniform dispersibility for more than 20 days at 4°C and ensures an advanced effect in that it increases easiness in the manufacture and storage stability of the suspension.

On the other hand, the "sinking rate" as used herein refers to a rate and degree at which particles settle in a fluid, and in the case of suspensions, a certain level of sedimentation must be satisfied (if 1 is the maximum, 0.9 or higher is required) in order to be approved for sale as a drug. The nanosuspension according to the present disclosure is very excellent in the sinking rate by using a vehicle solution that includes hydroxypropyl methylcellulose (HPMC), TPGS or dioctyl sodium sulfosuccinate (DOSS), and potassium sorbate that are suitable for the progerinin drug.

In addition, the pH of the suspension of the present disclosure may be 5.5 to 8.5, preferably 6.0 to 7.0.

In addition, the nanosuspension may have a viscosity of 2 to 8.5 mPa·s, preferably 7 to 8.5 mPa·s.

The nanosuspension of the present disclosure may further include one or more components selected from the group consisting of sweeteners, preservatives, flavors, pH adjusters, and colorants.

The term "sweetener" as used herein refers to a food additive that has (produces) a sweetness but generally a lower food caloric value, including those that are both natural and synthetic. The sweetener may be applied to the present disclosure without limitation if it is generally available in the field of food and medicine.

As used herein, the term "preservative" refers to a pharmaceutically acceptable substance that prevents microbial proliferation or degradation by undesired chemical changes. The preservative has bactericidal and/or fungicidal or antioxidant properties, and in the present disclosure, it is desirable to use potassium sorbate (d) in an amount of 0.1 to 0.5 wt%, and more preferably 0.2 wt%. Within the above range, it is possible to enhance the shelf life without degrading the effectiveness of the drug. If it is added before milling, the content decreases, so it is preferable to complete the nanosuspension by post-addition after milling.

The term "flavor" or "aroma" as used herein includes flavoring ingredients or compositions commonly used in the food industry, whether it is natural or synthetic. This includes a single compound or mixture.

The term "pH adjuster" as used herein refers to an excipient used to adjust the pH of a suspension to a desired value, and may be, for example, but is not limited to, citric acid, sodium citrate, or ascorbic acid, and those commonly used in the technical field of the present disclosure may be used in the present disclosure without limitation.

The term "colorant" as used herein is included to make difference in the color of the suspension and may be used to prepare a suspension that has a desired color by being included in a formulation. If it is commonly used in the technical field of the present disclosure, it may be included in the present disclosure without limitation.

In addition, the present disclosure provides a method of preparing a nanosuspension including a poorly soluble decursin derivative drug with enhanced bioavailability and stability.

Specifically, as an example embodiment, a method of preparing a nanosuspension according to the present disclosure includes (i) mixing purified water with hydroxypropyl methylcellulose (HPMC) (b) and D-α-tocopherol polyethylene glycol succinate (TPGS) (c) to prepare a vehicle solution and then adding and mixing progerinin represented by the following Chemical Formula 1 so as to prepare a suspension; (ii) subjecting the suspension to wet type ball milling to prepare a nanosuspension; and (iii) mixing the nanosuspension with potassium sorbate (d) to finally prepare a nanosuspension with enhanced stability.

As another example embodiment, a method of preparing a nanosuspension according to the present disclosure includes (i) mixing purified water with hydroxypropyl cellulose (HPC) (b'); and dioctyl sodium sulfosuccinate (DOSS) (c'); to prepare a vehicle solution and then adding and mixing progerinin (a) represented by the following Chemical Formula 1 so as to prepare a suspension; (ii) subjecting the suspension to wet type ball milling to prepare a nanosuspension; and (iii) mixing the nanosuspension with potassium sorbate (d) to finally prepare a nanosuspension with enhanced stability.

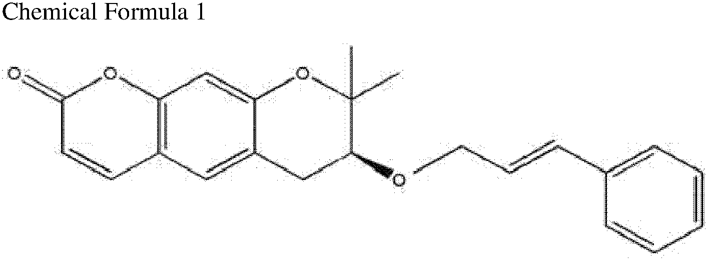

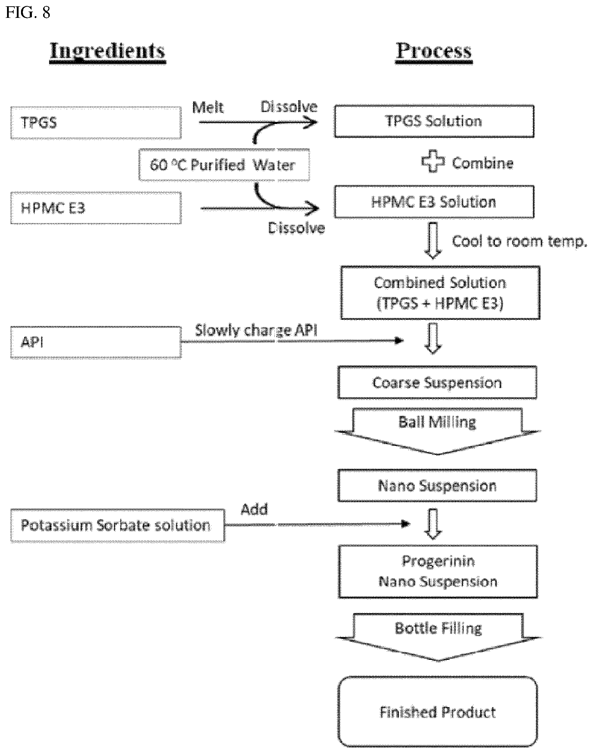

Since the role of each component and its dose are the same as mentioned above and it is redundant, further explanation is omitted. FIG. 8 is a flow diagram showing a method of preparing a nanosuspension including a poorly soluble decursin derivative drug with enhanced bioavailability and stability according to the present disclosure.

First, the step (i) in the present disclosure is to mix a micronized progerinin drug in a vehicle solution, specifically mixing purified water with hydroxypropyl methylcellulose (HPMC) or hydroxypropyl cellulose (HPC) (b'), which is a polymeric suspension; and D-α-tocopherol polyethylene glycol succinate (TPGS) (c) or dioctyl sodium sulfosuccinate (DOSS) (c'). Here, the purified water with a temperature of 50 to 70°C may be used, preferably a temperature of 60°C. The suspension (coarse suspension) thus mixed is then cooled to room temperature and then subjected to the next step to prepare the nanosuspension.

Next, the step (ii) is to prepare the nanosuspension by subjecting the suspension of the step (i) to the wet type ball milling. Here, dyno-mill may be used for the wet type ball milling. In terms of conditions for ball milling, it is preferable to carry out until the average particle diameter of the drug particles becomes 100 nm to 300 nm. More preferably, the average particle diameter is 150 nm to 250 nm, and even more preferably the average particle diameter is 200 nm. If it has the average particle diameter within the range, it is the most soluble while maximizing the bioavailability or bioabsorption efficiency of the drug.

Ball milling in the present disclosure refers to the wet type ball milling for preparation of nanosuspensions, which is an important step in the preparation of progerinin nanosuspensions. The selection of the suitable bead size, the crushing medium and API ratio in the chamber, and the temperature control of the crushing chamber are considered important.

Specifically, in the wet type ball milling in the preparation of nanosuspensions, the suspension of the step (i) and zirconia beads having the average particle diameter of 0.1 to 1 mm may be used, preferably 0.02 to 0.04 mm. In addition, the suspension of the step (i) and zirconia beads may be used in a volume ratio of 1:1 to 1:5, but if the average particle diameter (D50) of the drug is producible in 200 nm or less, it may be modified in accordance with the condition.

Next, the step (iii) is to mix the nanosuspension prepared in the step (ii) with potassium sorbate to finally prepare the nanosuspension with enhanced stability. The addition of potassium sorbate completely disperses the drug particles, thereby further enhancing the stability of the nanosuspension.

On the other hand, during the entire processes, a process of mixing and stirring poorly soluble drugs with solvents, water-soluble polymers, or surfactants is included, at which time a nanosuspension may be prepared at a mixing rate of 500 rpm to 1,000 rpm.

### Modes for Carrying Out the Invention

Hereinafter, to help the understanding of the present disclosure, example embodiments will be described in detail. However, the following example embodiments are provided to explain the present disclosure more completely to those with ordinary skill in the art and it is merely illustrative of the contents of the present disclosure, and therefore the scope of the present disclosure is not limited to the following example embodiments.

### <Example 1> Preparation of a lipid-based progerinin solution and limitations

In order to overcome the poor bioavailability of progerinin drugs, the inventors prepared a progerinin solution using a lipid-based preparation (monoolein:tricaprylin = 2:1) in a preclinical study. However, lipid-based preparations were determined to be unsuitable for use in clinical settings due to inadequate drug loading and high lipid intake expected with the proposed clinical dose. Limited attempts to produce amorphous solid dispersions (ASDs) have shown that there is a difficulty for the amorphous solid dispersions (ASDs) to be dispersed in aqueous media due to rapid crystallization. In addition, these lipid-based preparations are considered unsuitable for use in clinical settings due to inadequate drug loading and high lipid intake expected with the proposed clinical dose.

Accordingly, the inventors aimed to prepare progerinin, which is a poorly soluble drug, as a nanosuspension and embody the nanosuspension that is optimized for clinical use by selecting a vehicle composition that may enhance the stability of a drug.

The manufacturing process of nanosuspensions is thermodynamically unstable. Therefore, stabilizing agents such as polymers and surfactants are essential to retain a physically stable state. Of polymers, HPMC or HPC may be fixed onto the surface of drug particles, occupy adsorption sites, and prevent drug molecules from bonding into crystal lattices in solution, thus providing a mechanical barrier for crystallization. However, if the concentration of the polymer is insufficient, the crystals grow rapidly and agglomerate. As the concentration of polymers continues to increase, the size of the particles increases together due to the thick layer on the surface of the particles, and the diffusion between the solvent and semi-solvent is inhibited during the agglomeration process. In addition, as the concentration of the polymer increases, the osmotic pressure increases, resulting in an increase in the attraction among the colloidal particles. This leads to the growth of particles. On the other hand, surfactants are adsorbed at the solid-liquid interface to reduce the surface tension of the interface and increase the nucleation rate, which leads to an initial reduction in the particle size. Furthermore, the adsorption of the surfactant reduces hydrophobic interactions and coagulation, making resveratrol particles less hydrophobic and the particle growth reduced. In particular, when SLS, an ionic surfactant, is adsorbed onto the particle surface, the particle surface becomes negatively charged. This increases the repulsive force between the particles to increase the energy barrier, thereby preventing growth and agglomeration of the particles. As a result, the addition of a stabilizing agent at an appropriate concentration may lower the excessively high surface energy of the nanoparticles being manufactured. Furthermore, the size of the drug particles in the nanosuspension is an important factor in determining the bioabsorption efficiency.

Therefore, the inventors have determined that the selection of stabilizing agents, such as polymers and surfactants, as well as particle size in the suspension are important factors in the preparation of nanosuspensions of poorly soluble drugs. First, since the size of the drug particles in the drug is an important factor in determining the bioabsorption efficiency, the average particle diameter of the progerinin drug (SLC-D011) with excellent bioabsorption efficiency was chosen, and polymers, surfactants, and preservatives for the production of nanosuspensions were optimized.

### <1-1> Determination of average particle diameter (D50) of progerinin drug

As mentioned above, the size of the drug particles in the drug is an important factor in determining the bioabsorption efficiency. Therefore, in order to design a diameter size that is highly bioabsorbable for the progerinin drug, the inventors used mice to analyze pharmacokinetics (PK) of compounds. Specifically, a microsuspension including micro-sized progerinin (D 50 = 1.5 mm), a nanosuspenstion 1 (Nano suspenstion 1 = 200nm (D=50)) and a nanosuspenstion 2 (Nano suspenstion 2 = 350 nm (D=50)) including nano-sized progerinin were prepared. Here, the microsuspension was prepared using Vivapur power, and the two nanosuspensions were prepared using zirconia beads. The suspension prepared as described above was administered orally to mice at doses of 10 mg/kg, 30 mg/kg, and 100 mg/kg, and results were checked by analyzing the pharmacokinetics (PK) of the suspension in vivo (FIG. 1).

As a result of FIG. 1, it was determined that the bioabsorption of the drug is satisfied when the average particle size is 200 nm or less. Therefore, the inventors decided to develop in a suspension formulation including drug nanoparticles with an average particle diameter of 200 nm or less.

In order to devise a manufacturing method that can satisfy these formulations, the present inventors performed a process using hot melt extrusion, spray drying, ultra-high pressure homogenizer, and wet type ball milling using beads and attempted to select the most suitable method for preparing the progerinin nanosuspension among them.

### <1-2> Preparation of amorphous solid dispersions using hot melt extrusion (HME) process

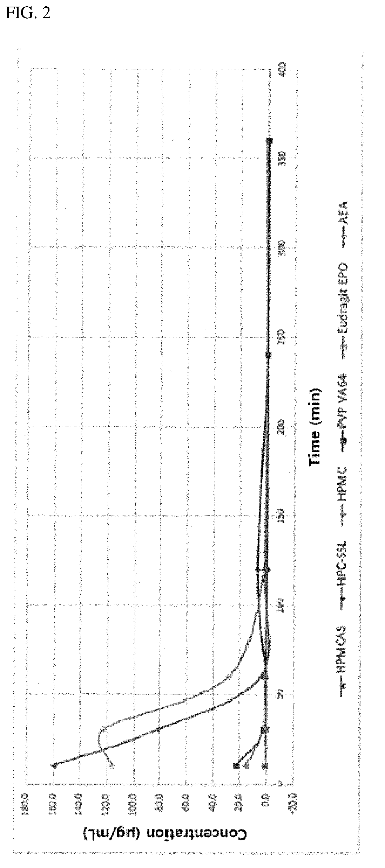

First, solid dispersions for various polymers (HPMCAS, HPC, HPMC, PVP VA64, Eudragit EPO, AEA) were prepared using the hot melt extrusion method. Here, the ratio of drug to polymer was set to 1:3 to prepare the mixture. Solubility tests were performed on a solid dispersion equivalent to 100 mg of progerinin with 300 mL of pH 1.2 buffer (Eudragit EPO, AEA) or 300 mL of pH 6.8 buffer (HPMCAS, HPC, HPMC, PVP VA64) as an eluate (FIGS. 2 to 4). The elution conditions are as follows: paddle method, temperature: 37°C, rotation rate: 150 rpm

As a result of FIG. 2, it was found that the solubility was improved the highest when HPMCAS was used as a polymer.

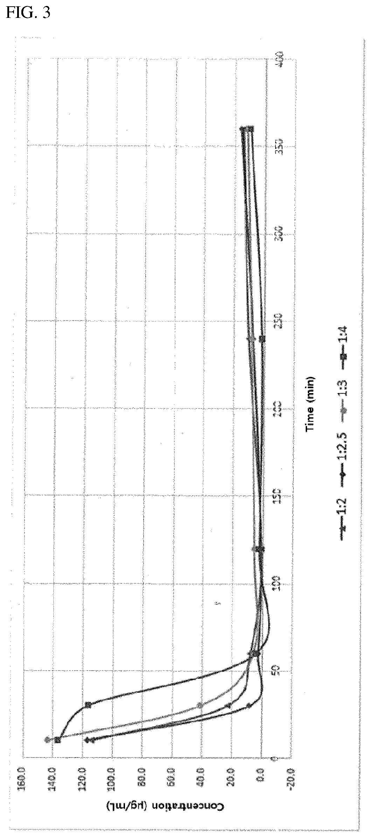

Next, the HPMCAS with the most improved solubility was re-selected and the solid dispersion was prepared in various ratios (1:2, 1:2.5, 1:3, 1:4). Solubility test was conducted on a solid dispersion equivalent to 100 mg of progerinin with 300 mL of pH 6.8 buffer as an eluate (FIG. 3). The elution conditions are as follows: paddle method, temperature: 37°C, rotation rate: 150 rpm

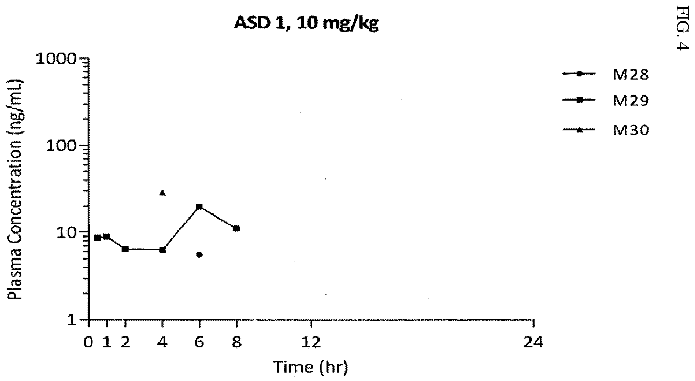

As a result of FIG. 3, it was identified that the solubility was highest when the ratio of the drug to HPMCAS was 1:3. However, in fact, it was possible to obtain a light-yellow powder form through the HME method and it was found that it remained amorphous in a solid form. In addition, as a result of FIG. 4, the powder obtained by the HME method immediately settled by precipitation after contact with water, and when bioabsorption using mice was observed, there was almost no absorption into vivo when administered orally.

Therefore, it was determined that progerinin, which is the main component of the current drug, could not be developed in a formulation by the hot melt extrusion process.

### <1-3> Preparation of amorphous solid dispersions via spray drying process

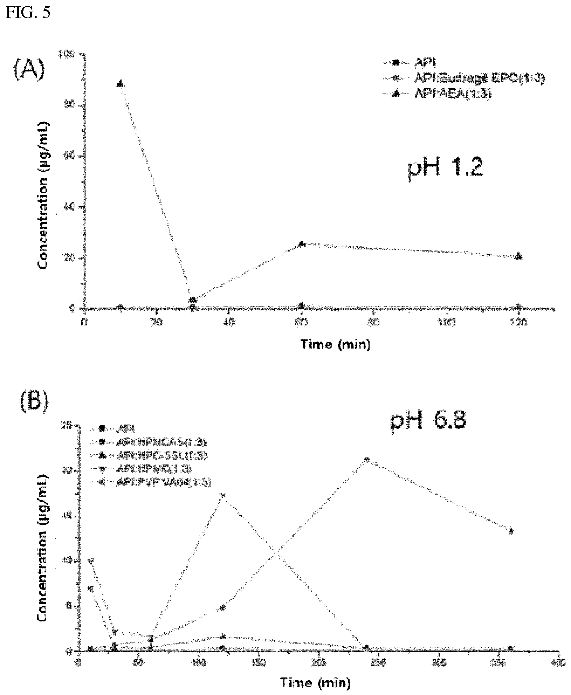

The polymer of the Example <1-2> was used, but an amorphous solid dispersion was prepared using the spray drying process. Solid dispersions for various polymers (HPMCAS, HPC, HPMC, PVP VA64, Eudragit EPO, AEA) were prepared via the spray drying process. At this time, the mixture was prepared by setting a ratio of drug and polymer to 1:3. Solubility test was conducted on a solid dispersion equivalent to 100 mg of progerinin with 300 mL of pH 1.2 buffer (Eudragit EPO, AEA) and 300 mL of pH 6.8 buffer (HPMCAS, HPC, HPMC, PVP VA64) as an eluate, respectively (FIG. 5). The elution conditions are as follows: paddle method, temperature: 37°C, rotation rate: 150 rpm

As a result of FIG. 5, it was found that the solubility of the polymer was improved the most at pH 1.2 and 6.8 when HPMCAS was used as the polymer in the same way as the solid dispersant prepared by the hot melt extrusion process.

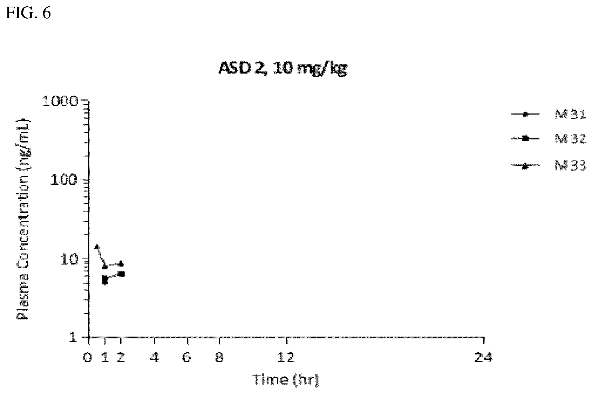

However, when bioabsorption (PK analysis) using mice was identified, absorption into vivo barely took place when administered orally, similar to HME (FIG. 6). In addition, the stability of other polymers under high temperature and high humidity for 2 weeks showed unsatisfactory results as the recrystallization of progerinin particles was observed (Table 1).

**TABLE 1**

| SD formu lation | Initial purity (%) | Storage condition | After 1 week | | | | | After 2 weeks | | | |
|---|---|---|---|---|---|---|---|---|---|---|---|
| | | | Appea rance | Tg by mDS C (°C) | XRPD | HPLC results | | XRPD | Tg by mDS C (°C) | HPLC results | |
| | | | | | | Drug load (%) | Purity (%) | | | Drug load (%) | Purity (%) |
| Crysta lline API | 99.85 | 25°C /60%RH, open | White powde r | N/A | Unchan ged | N/A | 99.85 | Unchan ged | N/A | N/A | 99.85 |
| | | 40°C /75%RH, open | White powde r | N/A | Unchan ged | N/A | 99.85 | Unchan ged | N/A | N/A | 99.85 |
| PVP VA64 | 96.36 | 25°C /60%RH, open | White powde r | 79.19 | Amorp hous | 19.9 | 96.56 | Amorp hous | 83.14 | 25.6 | 96.27 |
| | | 40°C /75%RH, open | White powde r | N/A | Crystall ine | 21.3 | 96.36 | Crystall ine | N/A | 22.5 | 96.39 |
| HPM C ASLF | 92.63 | 25°C /60%RH, open | White powde r | 67.25 | Amorp hous | 20.8 | 92.36 | Amorp hous | 67.82 | 22.5 | 92.04 |
| | | 40°C /75%RH, open | White powde r | N/A | Crystall ine | 21.7 | 91.53 | Crystall ine | N/A | 23.4 | 91.62 |
| HPM C E3 | 80.72 | 25°C /60%RH, open | White powde r | 98.75 | Amorp hous | 18.0 | 79.41 | Amorp hous | 96.50 | 17.1 | 79.65 |
| | | 40°C/75 %RH, open | White powde r | N/A | Crystall ine | 18.2 | 78.60 | Crystall ine | N/A | 16.2 | 77.45 |

Therefore, it was determined that progerinin, which is the main component of the current drug, could not be developed in a formulation by the spray drying method.

### <1-4> Preparation of nanosuspensions using ultra-high pressure homogenizers

The inventors have prepared the nanosuspensions using an ultra-high pressure homogenizer as an alternative method to prepare the nanosuspension. Specifically, polymer (HPMC 3cp) and surfactant (TPGS) were dissolved in a 100 mL beaker containing distilled water, and then the drug (SLC-D011) was weighed and added to prepare the suspension by stirring for 2 hours. The nanosuspension was prepared by injecting the suspension into an ultra-high pressure homogenizer (pressure: 40000 psi, 1 hour).

**TABLE 2**

| | Initial | 3 Day |
|---|---|---|
| Z-average (nm) | 338.6 | 354.6 |
| PI | 0.076 | 0.278 |
| D (90%) (nm) | 517.1 | 893.9 |

Table 2 is a measurement of the particle diameter of the drug, identifying that the size of the particles increased after 3 days. Therefore, the preparation of nanosuspensions using ultra-high pressure homogenizers had the limitation that the particles increase over time. Again, this is not a suitable method for the preparation of progerinin nanosuspensions.

### <1-5> Preparation of nanosuspensions via wet type ball milling with beads

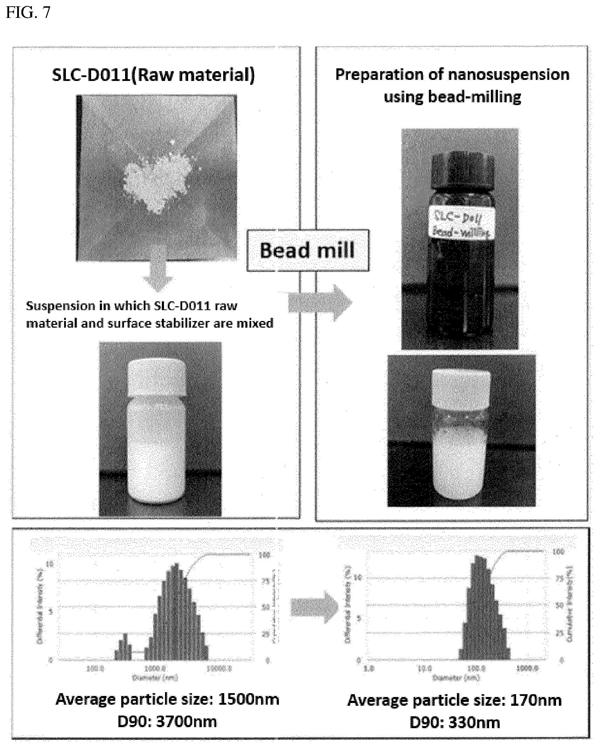

As an alternative method, the inventors prepared a nanosuspension using wet type ball milling using beads. Specifically, polymer (HPMC 3cp) and surfactant (TPGS) were dissolved in a 100 mL beaker containing distilled water, and then progerinin was weighed and added to prepare the suspension by stirring for 2 hours. Tubes with beads and suspensions added were mounted on DeltaVita and Netzsch (Zentrimix 380R) devices to prepare the nanosuspension (rate: 1200 rpm, temperature: -10°C, 6 hours). FIG. 7 shows the appearance (top) of the nanosuspension prepared by the preparation method and results (bottom) of measuring the average particle diameter.

As a result of FIG. 7, it was possible to prepare the nanosuspension including drug nanoparticles with an average particle diameter of 170 nm. In terms of the stability of nanosuspensions, it was found that the solubility was more excellent when the average particle diameter of the drug particles is 200 nm or less, and that the nanoparticles have a larger surface area and are more easily dissolved, thus maximizing the bioavailability.

Accordingly, the inventors determined that it was necessary to select a vehicle composition that is suitable for the preparation of nanosuspensions with an average particle diameter of 200 nm or less while preparing nanosuspensions using wet type ball milling using beads, and sought to select optimized polymers, surface stabilizing agents such as surfactants, and preservatives.

### <Example 2> Selection of optimized vehicle solutions

### <2-1> Preparation of progerinin nanosuspensions

### a) Preparation of suspensions (Step (i))

TPGS solution is prepared by dissolving TGPS in 60°C warm water and then cooling to room temperature. HPMC E3 solution is prepared by dissolving HPMC E3 in 60°C warm water and then cooling it to room temperature. Then the two solutions are stirred and mixed thoroughly. To prepare the suspension, progerinin as an active pharmaceutical ingredient (API) is filled in a slowly mixed solution with continuous stirring.

### b) Nano milling (Step (ii))

The parameters of the Agitator Bead Mill, a mixing and wet grinding equipment, are set appropriately to start the wet type ball milling, and a hydrostatic pump is adjusted to have the suspension circulated. The suspension escaping the Agitator Bead Mill is recirculated back into the suspension vessel while maintaining a preset value below 35°C. Wet type ball milling continues with a dyno-mill until the drug particles achieve PSD (D50 ≤ 200 nm). When the target PSD (D50≤200nm) is accomplished, the pump is turned off, and the milling suspension line is separated from the suspension vessel.

### c) Preparation of nanosuspensions with enhanced stability (Step (iii))

The suspension obtained by nano-milling is thoroughly mixed by adding potassium sorbate as a preservative. It is filled in a sterile glass jar. The bottle filling is carried out with 100% visual inspection and checking of the filling weight, after which the bottle is sealed with a rubber finish and an aluminum flip-off seal.

### <2-2> Screening of optimized vehicle solutions

The oral progerinin nanosuspension was prepared via the method in Example <2-1>. Specifically, Prototype 1 consists of 10.0 wt% of progerinin, 3.0 wt% of HPMC E3, 1.0 wt% of surfactant TPGS, and 0.2 wt% of potassium sorbate, and a suspension was prepared by mixing TPGS and HPMC solutions and then adding progerinin. The mixture prepared as described above was then mixed with 0.3 mm VHD ZrO beads in a volume ratio of 1:1 to 5, and the ball milling was performed. The temperature of the vessel was closely monitored during the preparation process. Furthermore, during milling, the particle size distribution was monitored until the target average particle size reached D50 ≤ 200 nm. Prototypes 2 to 5 were also prepared in the same way. The particle diameter, zeta potential, drug content, flowability, and viscosity of the nanosuspension prepared as described above were measured in the following methods.
(1) Measurement of particle diameter (D50): Approximately 2 µL of nanosuspension was dispersed in 1 mL of water and put into a sample tube, followed by measurement with the Zeta Potential & Particle Sizer (ZPPS) (Nicomp 380/ZLS, Nicomp), which is used for aqueous suspensions.
(2) Measurement of zeta potential: It is measurement of the electrical charge of the particle surface, indicating the physical stability of the colloidal system. Zeta potential was measured using the Laser-Doppler method.
(3) Measurement of drug content: The content of progerinin for the total weight of the nanosuspension was measured.
(4) Measurement of dispersibility: Dispersibility was evaluated by visual observation depending on the precipitation of the suspension.
(5) Viscosity measurement: Measurement was performed at a torsional moment of 32.9%, temperature of 25°C, probe rotation rate of 200 rpm, shear force of 13.03 dyne/cm², and shear rate of 264.0 S⁻¹ for 1 minute using a BROOKFIELD viscometer (TC-550MX-230).

**TABLE 3**

| Category | Prototype 1 | Prototype 2 | Prototype 3 | Prototype 4 | Prototype 5 |
|---|---|---|---|---|---|
| Composition (wt%) | HPMC E3 3%/ TPGS 1%/ Potassium sorbate 0.2% | HPMC E3 1%/TPGS 0.25%/ Potassium Sorbate 0.2% | HPC (SL) 1%/ DOSS 0.25%/ Potassium Sorbate 0.2% | HPC (SL) 1%/ Polysorbate 80 0.25%/ Potassium Sorbate 0.2% | PVP K30 1%/ Poloxamer 188 0.25%/ Benzoic acid 0.2% |
| D50 (Unit: nm) | 165.8 | 235.2 | 146.3 | 191.4 | N/A |
| D90 (Unit: nm) | 281.8 | 414.4 | 260.8 | 342.5 | N/A |
| PDI | 0.171 | 0.195 | 0.203 | 0.206 | N/A |
| Zete potential (Unit: mV) | -2.07 | N/A | -17.76 | -5.84 | N/A |
| API concentration of suspension (Unit: mg/mL) | 100.9 | N/A | 107.6 | N/A | N/A |
| Purity of suspension (Unit: %) | 99.9 | N/A | 99.9 | N/A | N/A |
| Impurity of suspension (Unit: %) | - | N/A | - | N/A | N/A |
| API concentration of supernatant (Unit: mg/mL) | 1.5 | N/A | 2.4 | N/A | N/A |
| Purity of supernatant (Unit: %) | 95.4 | N/A | 99.9 | N/A | N/A |
| Impurity of supernatant, (Unit: %) | 4.538 (RRT 0.963) | N/A | - | N/A | N/A |
| Flowability | good | good | good | good | Very poor |
| Viscosity (Unit: mPa·s) | 7.72 | N/A | 2.25 | 4.28 | N/A |

From Table 3, it was found that the average particle diameters (D50) of the drug particles of prototype 1 and prototype 3 were 165.8 nm and 146.3 nm, respectively with the average particle diameter of 200 nm or less satisfied, and the drug content had a value higher than 95% without the detection of impurities. Therefore, it was possible to determine that prototypes 1 and 3 are the most suitable vehicle compositions in terms of dispersibility, stability, and effectiveness of the drug. On the other hand, due to limitations in terms of effectiveness of the drug, the prototype 4 was not selected as a suitable vehicle composition. In the case of prototype 2, the tendency that particles increase over time, made it an unsuitable candidate. In the case of Prototype 5, the formation of nanosuspensions was not possible at all.

### <Example 3> Preparation of suspensions using roller mixer and dyno-mill and comparison of physical properties

<3-1> Preparation of suspensions using roller mixer

Using a roller mixer, it was intended to prepare a nanosuspension of prototype 1 at a target concentration of 100 mg/g. Specifically, the nanosuspension of prototype 1 was prepared, and the prepared nanosuspension was mixed with the zirconia beads shown in Table 2 below in a 1:1 weight ratio. Furthermore, as shown in Table 4, the PSD was monitored daily to observe the tendency of size reduction.

**TABLE 4**

| Grinding media | Milling time (day) | D50(nm) | D90(nm) | PDI |
|---|---|---|---|---|
| 0.8 mm beads | 1 | 786.9 | 1360.1 | 0.182 |
| | 2 | 513.8 | 990.2 | 0.262 |
| | 3 | 451.5 | 806.8 | 0.205 |
| | 4 | 394.4 | 671.4 | 0.172 |
| | 5 | 355.8 | 672.8 | 0.247 |
| 0.5 mm beads | 6 | 345.3 | 570.7 | 0.154 |
| | 7 | 335.1 | 533.0 | 0.131 |
| 0.2 mm beads | 8 | 308.0 | 505.7 | 0.150 |

The results in Table 4 show that after 8 days of milling, the particle size stagnates at D90 = 500 nm without a further decrease. Therefore, in the case of roller mixers, it was determined that it would be difficult to prepare nanosuspensions including drug particles below 200 nm.

### <3-2> Preparation of suspensions using dyno-mill and evaluation of properties thereof

Based on the results from <3-2> above, the inventors used a dyno-mill (5 g API scale) to prepare a nanosuspension of prototype 1 at a target concentration of 100 mg/g. Specifically, loaded in a dyno-mill milling chamber were 45 mL of 0.5 mm zirconia beads. The prepared nanosuspension of prototype 1 (5 g, 10% w/w HPMC E3, 3%/TPGS 1%) was filled in the milling chamber from the top. The device was operated for 5 hours at 2,200 rpm with a -19°C circulating cooling liquid. White nanosuspension (FR00497-3-190911-100) was collected as the final nanosuspension.

In addition, collection was performed in the same method as above in an attempt to prepare a nanosuspension of 6 mg/g prototype 1 (FR00497-3-190910-6) and a nanosuspension of 20 mg/g prototype 1 (FR00497-3-190910-20). The three nanosuspensions thus collected were identified for appearance, PSD, HPLC, and syringeability, the results of which are shown in Table 5 below.

**TABLE 5**

| Batch No. | FR00497-3-190911-100 | FR00497-3-190910-20 | FR00497-3-190910-6 |
|---|---|---|---|
| Target conc. (mg/g) | 100 | 20 | 6 |
| Measured conc. (mg/g) | 98.9 | 19.3 | 5.8 |
| Volume (mL) | 7 | | |
| Appearance | White suspension | | |
| PDI. | 0.226 | 0.204 | 0.207 |
| Particle size (D50, nm) | 211.0 | 222.9 | 209.6 |
| Particle size (D90, nm) | 387.9 | 397.9 | 375.5 |
| Syringeability | Easily pass | | |

As a result of Table 5, it was found that the nanosuspension of prototype 1 satisfied all of its physical properties such as particle size and syringeability. Additionally, the low-temperature stability of the three nanosuspensions at 4°C was observed for 2 weeks. Specifically, the stability was evaluated by measuring the particle size to determine whether the average particle diameter (D50) is 200 nm or less, and the syringeability was evaluated by checking whether the nanosuspension was easy to pass through the 20 GA syringe, the results of which are shown in Table 6 below.

**TABLE 6**

| Batch No. | FR00497-3-190911-100 | | FR00497-3-190910-20 | | FR00497-3-190910-6 | |
|---|---|---|---|---|---|---|
| Time | Initial | 4°C (for 2 weeks) | Initial | 4°C (for 2 weeks) | Initial | 4°C (for 2 weeks) |
| Appearance | White homogeneous suspension | | | | | |
| PDI. | 0.226 | 0.251 | 0.204 | 0.166 | 0.207 | 0.239 |
| PSD (D50, nm) | 211.0 | 195.0 | 222.9 | 248.8 | 209.6 | 200.9 |
| PSD (D90, nm) | 387.9 | 370.5 | 397.9 | 419.2 | 375.5 | 375.9 |
| Syringeability | Easily pass | | | | | |

As a result of FIG. 6, it was found that all three nanosuspensions had stability at a low temperature of 4°C without significant changes in the particle diameter, and they had excellent syringeability. Herein, the condition for the average particle diameter (D50) of 200 nm or less is the most ideally required in terms of the dispersion stability of the particle and the bioavailability efficiency of the progerinin drug.

### <3-3> Preparation of nanosuspensions by scale-up

*The present inventors sought to prepare a nanosuspension with a target concentration of 100 mg/g using a dyno-mill for a 30 g API scale for the preparation by scale up. Specifically, loaded in the dyno-mill chamber were 45 mL of 0.3 mm zirconia beads. The 30 g of prepared progerinin is first suspended in a vehicle solution to prepare a suspension (30 g, 10% w/w in the vehicle HPMC E3 3%/TPGS 1% in water), and then the suspension is filled into the milling chamber from the top. At a low temperature of -19°C, the device with circulating cooling liquid was operated for 20 hours at 2,200 to 2,500 rpm. The PSD was monitored over time as shown in Table 7, and finally the white nanosuspension was collected. Furthermore, the stability was monitored by observing changes in particle size of the obtained white nanosuspension for 20 to 70 days under refrigerated conditions (Table 8).

**TABLE 7**

| Milling time/h | D50(nm) | D90(nm) | PDI |
|---|---|---|---|
| 3 | 264.4 | 513.0 | 0.267 |
| 6 | 201.2 | 362.4 | 0.211 |
| 8 | 187.5 | 328.6 | 0.192 |
| 11 | 174.3 | 307.0 | 0.195 |
| 13 | 170.8 | 309.2 | 0.214 |
| 16 | 162.7 | 297.1 | 0.221 |
| 18 | 166.3 | 294.1 | 0.198 |
| 20 | 177.3 | 337.4 | 0.252 |

The results of Table 7 show that even if the nanosuspension is prepared by scale-up using a dyno-mill, it is possible to successfully prepare progerinin drug particles with an average particle diameter (D50) of 200 nm or less.

**TABLE 8**

| Time | Initial | 4°C (20 days) | 4°C (70 days) |
|---|---|---|---|
| Target conc. (mg/g) | 100 | | |
| Measured conc. (mg/g) | 99.7 | 95.2 | 97.7 |
| Supernatant conc. (mg/g) | - | 0.46 | - |
| Purity (%) | 98.3 | 99.8 | 99.0 |
| PDI | 0.215 | 0.223 | 0.247 |
| Particle size (D50, nm) | 182.2 | 192.4 | 178.0 |
| Particle size (D90, nm) | 330.1 | 352.2 | 336.6 |
| Volume (mL) | 125 | | |
| Appearance | White homogeneous suspension | | |
| Syringeability | Easily pass | | |

The results of Table 8 show that the nanosuspension according to the present disclosure continues to maintain the stability for more than 20 days under refrigeration conditions, specifically for 20 to 70 days.

### <Example 4> Selection of optimized excipients

To assess compatibility with excipients, progerinin was mixed with HPMC E3, TPGS, and potassium sorbate in a ratio of 1:10, and the effectiveness of the drug (API) components was measured under 40°C/75% RH condition over 2 weeks and shown in Table 9 and Table 10.

**TABLE 9**

| Binary Mixture 1:10 ratio | Condition | Duration | RRT=0.43 | RRT=0.9 | API(% Area) | RRT=1.02 |
|---|---|---|---|---|---|---|
| API control | Initial | 0 | -- | -- | 99.94 | 0.06 |
| | 40°C/75% RH | 1 week | -- | -- | 99.94 | 0.06 |
| | | 2 weeks | -- | -- | 99.95 | 0.05 |
| API+HPMC E3 | Initial | 0 day | -- | -- | 99.95 | 0.05 |
| | 40°C/75% RH | week | -- | -- | 99.94 | 0.06 |
| | | 2 weeks | -- | -- | 99.95 | 0.05 |
| API+TPGS | Initial | 0 day | -- | -- | 99.96 | -- |
| | 40°C/75% RH | week | -- | 0.06 | 99.91 | -- |
| | | 2 weeks | -- | 0.1 | 99.85 | -- |
| API+potassium | Initial | 0 day | -- | -- | 99.94 | 0.06 |

**TABLE 10**

| Binary Mixture 1:10 ratio | Condition | Duration | RRT=0.43 | RRT=0.9 | API(% Area) | RRT=1.02 |
|---|---|---|---|---|---|---|
| sorbate | 40°C/75% RH | 1 week | -- | -- | 99.95 | 0.05 |
| | | 2 weeks | -- | -- | 99.95 | 0.05 |

| | | | | | | |
|---|---|---|---|---|---|---|
| API:Active Pharmaceutical Ingredient (SLC-D001) | | | | | | |

The results of Tables 9 and 10 show that, even if HPMC E3, TPGS, and potassium sorbate are added to the progerinin drug, there was no effect on the drug. In particular, in the case of preservatives, the content decreases when added before milling, such that it is preferable to complete it by post-addition after milling. These preliminary excipient compatibility studies suggest that the drug has compatibility with excipients.

### <Example 5> Identification of stability

Stabilization studies of progerinin nanosuspensions were further conducted on prototypes 1 and 3, which showed excellent particle stability in the course of the vehicle solution selection. Specifically, evaluation was performed at 2 to 8°C, 40°C/RH 75% conditions, or 1.2 M lux exposure over a period of 3 weeks. The study on the stabilization of the progerinin nanosuspension was conducted at room temperature (25 ± 5°C, 60% RH) for 3 weeks. Characterization was conducted in relation to particle size and drug content of the progerinin nanosuspension. The results are shown in Tables 11 and 12.

**TABLE 11**

| Category | Prototype 1 HPMC E3 3%/ TPGS 1%/ Potassium sorbate 0.2% | | | | | | | |
|---|---|---|---|---|---|---|---|---|
| Condition | Initial | 2-8°C (1-week) | 2-8°C (2-week) | 2-8°C (3-week) | 40°C /RH75% (1-week) | 40°C /RH75% (2-week) | 40°C /RH75% (3-week) | 25°C.2MLux |
| D50(Unit: nm) | 165.8 | 208.3 | 208.0 | 200.0 | 194.0 | 194.2 | 195.9 | 186.3 |
| D90(Unit: nm) | 281.8 | 346.4 | 347.7 | 336.2 | 339.6 | 333.6 | 329.1 | 352.2 |
| PDI | 0.171 | 0.158 | 0.161 | 0.164 | 0.191 | 0.178 | 0.164 | 0.247 |
| Zete potential(Unit: mV) | -2.07 | N/A | N/A | N/A | N/A | N/A | N/A | N/A |
| API concentration of nanosuspension (Unit: mg/mL) | 100.9 | 108.3 | 103.5 | 99.8 | 103.1 | 99.7 | 97.6 | 103.9 |
| Purity of suspension (Unit: %) | 99.9 | 99.9 | 99.9 | 99.5 | 99.9 | 99.9 | 99.4 | 99.5 |
| Impurity of suspension (Unit: %) | - | - | - | - | - | - | - | 0.468 (RRT 1.015) |
| API concentration of supernatant (Unit: mg/mL) | 1.5 | 1.4 | 1.4 | 2.0 | 0.834 | 0.62 | 1.4 | 0.821 |
| Purity of supernatant (Unit: %**) | 99.9 | 99.9 | 99.9 | 99.5 | 99.9 | 99.9 | 95.9 | 99.9 |
| Impurity of supernatant (Unit: %) | - | - | - | RRT0.66 0.45% | - | - | RRT0.40 0.765; RRT0.43 1.6%; RRT0.52 1.7% | - |
| Flowability | good | good | good | good | good | good | good | good |
| Viscosity (Unit: mPa·s) | 7.72 | N/A | N/A | N/A | N/A | N/A | N/A | N/A |
| - N/A : No data because data cannot be measured | | | | | | | | |

**TABLE 12**

| Category | Prototype 3 HPMC (SL) 1%/ DOSS 0.25%/ Potassium Sorbate 0.2% | | | | | | | |
|---|---|---|---|---|---|---|---|---|
| Condition | Initial | 2-8 °C 1-week | 2-8 °C 2-week | 2-8 °C 3-week | 40 °C/ RH75% 1-week | 40 °C/ RH75% 2-week | 40 °C/ RH75% 3-week | 25 °C 1.2MLux |
| D50(Unit: nm) | 146.3 | 174.8 | 177.8 | 189.5 | 162.4 | 162.2 | 169.2 | 153.7 |
| D90(Unit: nm) | 260.8 | 321.3 | 330.6 | 345.3 | 287.5 | 288.7 | 302.0 | 270.4 |
| PDI | 0.203 | 0.226 | 0.234 | 0.219 | 0.199 | 0.202 | 0.204 | 0.194 |
| Zete potential(Unit: mV) | -17.76 | N/A | N/A | N/A | N/A | N/A | N/A | N/A |
| API concentration of nanosuspension (Unit: mg/mL) | 107.6 | 107.5 | 108.8 | 101.0 | 103.5 | 101.2 | 98.1 | 102.4 |
| Purity of suspension (Unit: %) | 99.9 | 99.9 | 99.9 | 99.9 | 99.9 | 99.9 | 99.9 | 99.4 |
| Impurity of suspension (Unit: %) | - | - | - | - | - | - | - | 0.597 (RRT 1.015) |
| API concentration of supernatant (Unit: mg/mL) | 2.4 | 1.3 | 1.4 | 2.0 | 0.31 | 1.3 | N/A | 0.29 |
| Purity of supernatant (Unit: %) | 99.9 | 99.9 | 99.9 | 99.9 | 99.9 | 99.9 | 99.9 | 99.9 |
| Impurity of supernatant, (Unit: %) | - | - | - | - | - | - | - | - |
| Flowability | good | good | good | good | good | good | good | good |
| Viscosity, (Unit: mPa·s) | 2.25 | N/A | N/A | N/A | N/A | N/A | N/A | N/A |
| - N/A : No data because data cannot be measured | | | | | | | | |

The results of Table 11 and Table 12 show that even under low temperatures or harsh conditions, the average particle size was 200 nm or less, ensuring stability of the nanodrug particles for more than 3 weeks.

### <Example 6> Identification of stability of optimized progerinin nanosuspension in simulated intestinal fluids (SIF) and simulated gastric fluids (SGF)

Preparation was performed in the same method as in Example <2-1>, but the nanosuspension of prototype 1 was prepared with the components and content in Table 13 below. The dispersion stability in the simulated intestinal fluid (SIF) and simulated gastric fluid (SGF) was checked for the nanosuspensions prepared as described above. The specific experimental method is as follows.

**TABLE 13**

| **Component** | **Function** | **Unit Quantity (per bottle) (Unit: g)** | **Batch Quantity (per batch) (Unit: g)** | **Wt%** |
|---|---|---|---|---|
| SLC-D011 (Progerinin) | Active | 1.5 | 2,000.0 | 10 |
| HPMC E3 | Suspending agent | 0.45 | 600.0 | 3 |
| TPGS | Surfactant | 0.15 | 200.0 | 1 |
| Potassium Sorbate | Preservative | 0.03 | 40.0 | 0.2 |
| Purified water | Solvent | 12.87 | 17,160.0 | 85.8 |
| Total | | 15.0 | 20,000.0 | 100 |

### a) Simulated intestinal fluid (SIF)

Monobasic potassium phosphate (6.8 gm) and sodium hydroxide (0.616 gm) were added to a 1000 ml volume flask with 250 ml distilled water, then rotated until the above components are dissolved, and then another 700 ml of distilled water was added, followed by pH measurement. The pH was adjusted to pH 6.8 +/- 0.1 by adding 0.2N sodium hydroxide or 0.2N hydrochloric acid.

### b) Simulated gastric fluids (SGF)

Sodium chloride (2 gm), 750 ml of distilled water, and 7.0 ml of concentrated hydrochloric acid were added to a 1000 ml volume flask, followed by addition of 1000 ml distilled water and mixing of ingredients by rotation. The pH of this solution was set to 1.2.

### c) Preparation of the dispersion

A translucent HDPE vial (25 ml) with an HDPE lid was added with a nanosuspension of prototype 1 in an appropriate amount. At this time, it was diluted in 15 ml of simulated gastric fluids or simulated intestinal fluids to reach a final concentration of progenin to 0.5 mg/ml. Then, after adding a dispersant, the formulation prepared above was shaken until it was completely dispersed. The vial was placed in an oil bath at 37°C until flocculation takes place. The size of the precipitated particles was measured using the Horiba-LA-910 particle analyzer. In general, the above substances were cultured in human gastric fluid on an empty stomach for approximately 3 hours.

### d) Measurement of particle diameters

In the case of measuring beads coated with bead cores including insoluble particles, the weight of the bead cores was calculated in the SIF or SGF experiment, and the bead cores were dispersed in volumes such as SIF or SGF. 120 gm of distilled water was poured into the Horiba LA-910 chamber and drained until the device was empty. Afterwards, 120 gm of distilled water was poured, and the entire volume of the cultured formulation (at 15 ml of SGF or SIF) was poured into the Horiba LA-910 chamber and the average particle diameter was measured.

**TABLE 14**

| Test | Before dilution | Diluted in SGF (1V:9V) | Diluted in SIF (1V:9V) |
|---|---|---|---|
| Appearance | White homogeneous suspension | | |
| PDI. | 0.251 | 0.186 | 0.224 |
| PSD(D50, nm) | 195.0 | 218.5 | 212.5 |
| PSD(D90, nm) | 370.5 | 379.6 | 389.6 |

The results of Table 14 show that the progerinin drug (prototype 1) with the average particle diameter (D50) of 200 nm or less was maintained at 190 to 220 nm even in simulated intestinal fluids or simulated gastric fluids, and the dispersion stability and bioavailability of the drug were maintained.

## Claims

1. A nanosuspension comprising progerinin, the nanosuspension comprising:
(a) 1 to 10 wt% of a progerinin compound represented by the following Chemical Formula 1;
(b) 0.5 to 5 wt% of hydroxypropyl methylcellulose (HPMC) which is a polymeric suspension;
(c) 0.5 to 5 wt% of D-α-tocopherol polyethylene glycol succinate (TPGS) which is a solubility enhancer; and
(d) 0.1 to 0.5 wt% of potassium sorbate which is a preservative: 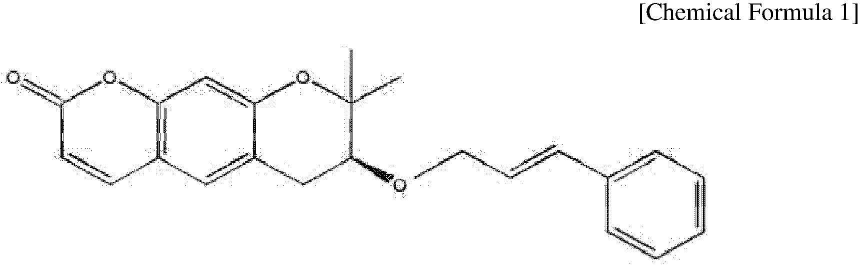 wherein the progerinin compound is drug particles formed via wet type ball milling in an average particle diameter (D50) of 100 nm to 300 nm.

2. A nanosuspension comprising progerinin, the nanosuspension comprising:
(a) 1 to 10 wt% of a progerinin compound represented by the following Chemical Formula 1;
(b') 0.5 to 5 wt% of hydroxypropyl cellulose (HPC) which is a polymeric suspension;
(c') 0.1 to 0.5 wt% of dioctyl sodium sulfosuccinate (DOSS) which is a surfactant; and
(d) 0.1 to 0.5 wt% of potassium sorbate which is a preservative: 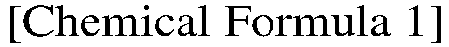 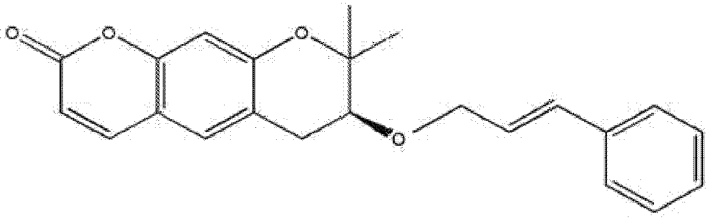
wherein the progerinin compound is drug particles formed via wet type ball milling in an average particle diameter (D50) of 100 nm to 300 nm.

3. The nanosuspension of claim 1 or claim 2, wherein an average particle diameter (D50) of the poorly soluble progerinin drug is 150 nm to 250 nm.

4. The nanosuspension of claim 1 or claim 2, wherein a viscosity of the nanosuspension is 2 to 8.5 mPa·s.

5. The nanosuspension of claim 1 or claim 2, wherein the nanosuspension has stability owing to uniform dispersibility of drug particles for more than 20 days under a temperature of 4°C.

6. A method of preparing a nanosuspension comprising progerinin, the method comprising:
(i) mixing purified water with hydroxypropyl methylcellulose (HPMC) (b) and D-α-tocopherol polyethylene glycol succinate (TPGS) (c) to prepare a vehicle solution and then adding and mixing progerinin (a) represented by the following Chemical Formula 1 so as to prepare a suspension;
(ii) subjecting the suspension to wet type ball milling to prepare a nanosuspension; and
(iii) mixing the nanosuspension with potassium sorbate (d) to finally prepare a nanosuspension with enhanced stability: 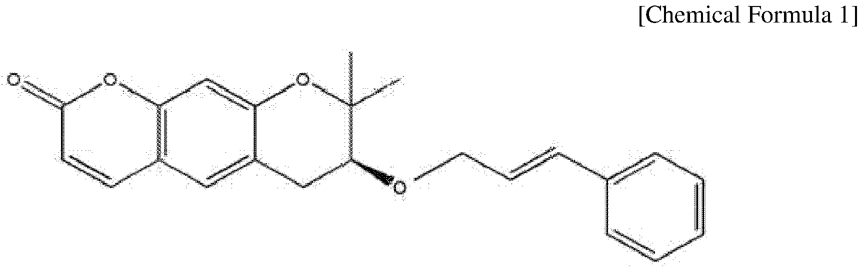 wherein the progerinin compound in the nanosuspension of (iii) is in the form of drug particles with an average particle diameter (D50) of 100 nm to 300 nm.

7. A method of preparing a nanosuspension comprising progerinin, the method comprising:
(i) mixing purified water with hydroxypropyl cellulose (HPC) (b'); and dioctyl sodium sulfosuccinate (DOSS) (c') to prepare a vehicle solution and then adding and mixing progerinin (a) represented by the following Chemical Formula 1 so as to prepare a suspension;
(ii) subjecting the suspension to wet type ball milling to prepare a nanosuspension; and
(iii) mixing the nanosuspension with potassium sorbate (d) to finally prepare a nanosuspension with enhanced stability: 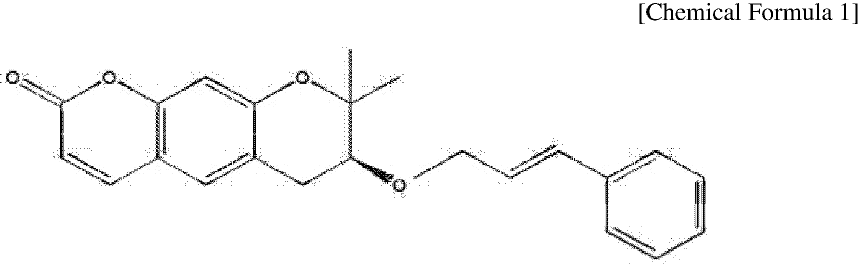 wherein the progerinin compound in the nanosuspension of (iii) is in the form of drug particles with an average particle diameter (D50) of 100 nm to 300 nm.

8. The method of claim 6 or claim 7, wherein a temperature of the purified water of (i) is 50 to 70°C.

9. The method of claim 6 or claim 7, wherein, when performing the wet type ball milling in (ii), the nanosuspension is prepared by mixing the suspension of (i) and zirconia beads in a volume ratio of 1:1 to 1:5 followed by milling.

10. The method of claim 6 or claim 7, wherein the nanosuspension of (iii) comprises progerinin drug nanoparticles with an average particle diameter of 150 nm to 250 nm.

## Patentansprüche

1. Progerinin umfassende Nanosuspension, wobei die Nanosuspension Folgendes umfasst:
(a) 1 bis 10 Gew.-% einer Progerinin-Verbindung, die durch die nachstehende chemische Formel 1 dargestellt ist;
(b) 0,5 bis 5 Gew.-% Hydroxylpropylmethylcellulose (HPMC), wobei es sich um eine polymere Suspension handelt;
(c) 0,5 bis 5 Gew.-% D-α-Tocopherol-Polyethylenglykol-Succinat (TPGS), bei dem es sich um einen Löslichkeitsverbesserer handelt; und
(d) 0,1 bis 0,5 Gew.-% Kaliumsorbat, bei dem es sich um ein Konservierungsmittel handelt; 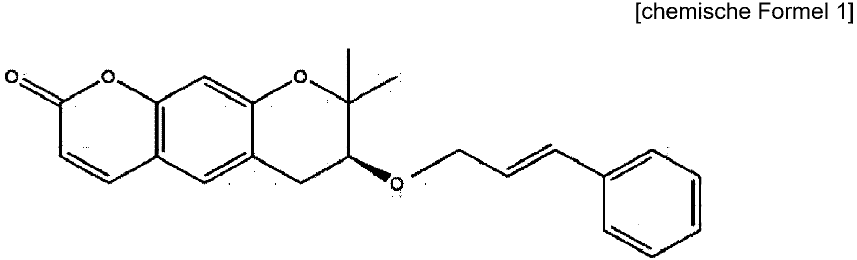 wobei die Progerinin-Verbindung in Form von Arzneimittel-Partikel vorliegt, die durch Nasskugelmahlen zu einem durchschnittlichen Partikeldurchmesser (D50) von 100 nm bis 300 nm gebildet werden.

2. Progerinin umfassende Nanosuspension, wobei die Nanosuspension Folgendes umfasst:
(a) 1 bis 10 Gew.-% einer Progerinin-Verbindung, die durch die nachstehende chemische Formel 1 dargestellt ist;
(b') 0,5 bis 5 Gew.-% Hydroxylpropylcellulose (HPC), wobei es sich um eine polymere Suspension handelt;
(c') 0,1 bis 0,5 Gew.-% Dioctylnatriumsulfosuccinat (DOSS), bei dem es sich um ein Tensid handelt; und
(d) 0,1 bis 0,5 Gew.-% Kaliumsorbat, bei dem es sich um ein Konservierungsmittel handelt; 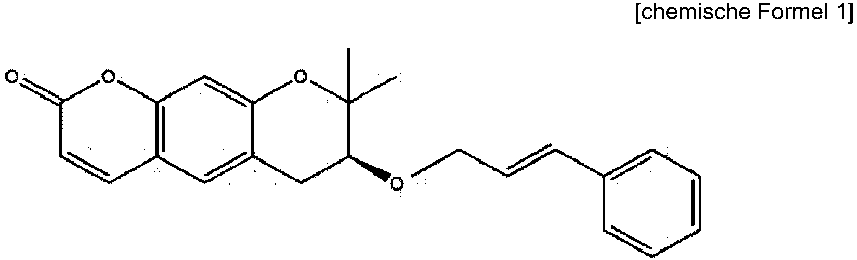
wobei die Progerinin-Verbindung in Form von Arzneimittel-Partikel vorliegt, die durch Nasskugelmahlen zu einem durchschnittlichen Partikeldurchmesser (D50) von 100 nm bis 300 nm gebildet werden.

3. Nanosuspension nach Anspruch 1 oder 2, wobei der durchschnittliche Partikeldurchmesser (D50) des schlecht löslichen Progerinin-Arzneimittels 150 nm bis 250 nm beträgt.

4. Nanosuspension nach Anspruch 1 oder Anspruch 2, wobei die Viskosität der Nano-suspension 2 bis 8,5 mPa·s beträgt.

5. Nanosuspension nach Anspruch 1 oder Anspruch 2, wobei die Nanosuspension eine Stabilität aufweist, die auf die gleichförmige Dispergierbarkeit von Arzneimittel-Partikel über mehr als 20 Tage bei einer Temperatur von 4 °C zurückzuführen ist.

6. Verfahren zur Herstellung einer Progerinin umfassenden Nanosuspension, wobei das Verfahren Folgendes umfasst:
(i) das Vermischen von gereinigtem Wasser mit Hydroxypropylmethylcellulose (HPMC) (b) und D-α-Tocopherol-Polyethylenglykol-Succinat (TPGS) (c), um eine Vehikel-Lösung herzustellen, und das anschließende Zusetzen und Einmischen von Progerinin (a), das durch die nachstehende chemische Formel 1 dargestellt ist, um eine Suspension herzustellen;
(ii) das Unterziehen der Suspension gegenüber Nasskugelmahlen, um eine Nano-suspension herzustellen; und
(iii) das Vermischen der Nanosuspension mit Kaliumsorbat (d), um schließlich eine Nanosuspension mit erhöhter Stabilität herzustellen; 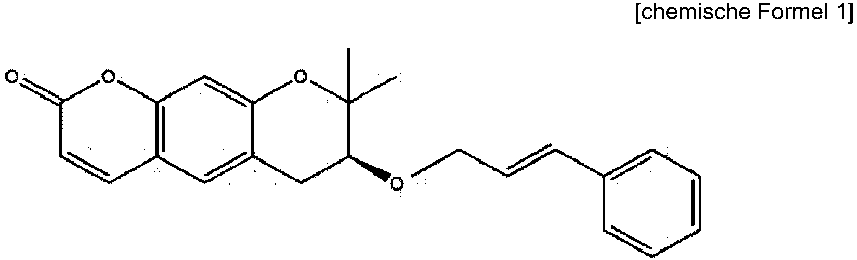 wobei die Progerinin-Verbindung in der Nanosuspension von (iii) in Form von Arzneimittel-Partikel mit einem durchschnittlichen Partikeldurchmesser (D50) von 100 nm bis 300 nm vorliegt.

7. Verfahren zur Herstellung einer Progerinin umfassenden Nanosuspension, wobei das Verfahren Folgendes umfasst:
(i) das Vermischen von gereinigtem Wasser mit Hydroxypropylcellulose (HPMC) (b'); und Dioctylnatriumsulfosuccinat (DOSS) (c'), um eine Vehikel-Lösung herzustellen, und das anschließende Zusetzen und Einmischen von Progerinin (a), das durch die nachstehende chemische Formel 1 dargestellt ist, um eine Suspension herzustellen;
(ii) das Unterziehen der Suspension gegenüber Nasskugelmahlen, um eine Nano-suspension herzustellen; und
(iii) das Vermischen der Nanosuspension mit Kaliumsorbat (d), um schließlich eine Nanosuspension mit erhöhter Stabilität herzustellen; 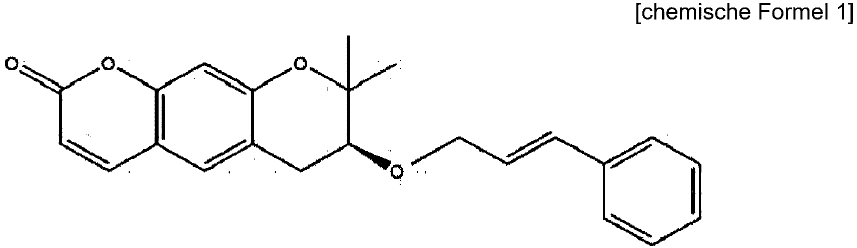 wobei die Progerinin-Verbindung in der Nanosuspension von (iii) in Form von Arzneimittel-Partikel mit einem durchschnittlichen Partikeldurchmesser (D50) von 100 nm bis 300 nm vorliegt.

8. Verfahren nach Anspruch 6 oder 7, wobei die Temperatur des gereinigten Wassers unter (i) 50 °C bis 70 °C beträgt.

9. Verfahren nach Anspruch 6 oder 7, wobei bei der Durchführung des Nasskugelmahlens unter (ii) die Nanosuspension durch Vermischen der Suspension aus (i) mit Zirconium-Kügelchen in einem Volumenverhältnis von 1:1 bis 1:5 gefolgt von Mahlen hergestellt wird.

10. Verfahren nach Anspruch 6 oder 7, wobei die Nanosuspension unter (iii) Progerinin-Arzneimittel-Nanopartikel mit einem durchschnittlichen Partikeldurchmesser von 150 bis 250 nm umfasst.

## Revendications

1. Nano-suspension comprenant de la progérine, la nano-suspension comprenant :
(a) de 1 à 10 % en poids d'un composé de progérine représenté par la formule chimique 1 suivante ;
(b) de 0,5 à 5 % en poids d'hydroxypropylméthylcellulose (HPMC) qui est une suspension polymère ;
(c) de 0,5 à 5 % en poids de succinate de D-α-tocophérol polyéthylène glycol (TPGS) qui est un activateur de solubilité ; et
(d) de 0,1 à 0,5 % en poids de sorbate de potassium qui est un conservateur : 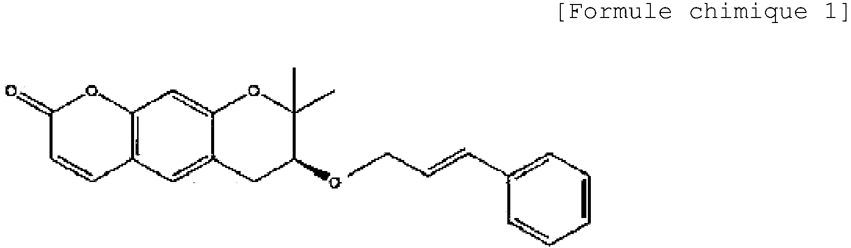 dans laquelle le composé de progérine est des particules de médicament formées par broyage à billes de type humide dans un diamètre de particule moyen (D50) de 100 nm à 300 nm.

2. Nano-suspension comprenant de la progérine, la nano-suspension comprenant :
(a) de 1 à 10 % en poids d'un composé de progérine représenté par la formule chimique 1 suivante ;
(b') de 0,5 à 5 % en poids d'hydroxypropylcellulose (HPC) qui est une suspension polymère ;
(c') de 0,1 à 0,5 % en poids de sulfosuccinate de dioctyle et de sodium (DOSS) qui est un tensioactif ; et
(d) de 0,1 à 0,5 % en poids de sorbate de potassium qui est un conservateur : 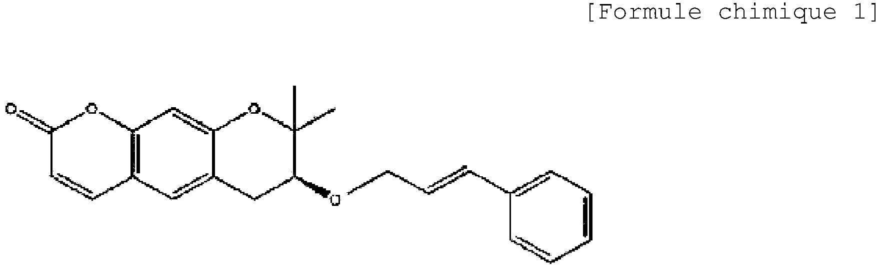 dans laquelle le composé de progérine est des particules de médicament formées par broyage à billes de type humide dans un diamètre de particule moyen (D50) de 100 nm à 300 nm.

3. Nano-suspension selon la revendication 1 ou la revendication 2, dans laquelle un diamètre de particule moyen (D50) du médicament de progérine faiblement soluble est de 150 nm à 250 nm.

4. Nano-suspension selon la revendication 1 ou la revendication 2, dans laquelle une viscosité de la nano-suspension est de 2 à 8,5 mPa-s.

5. Nano-suspension selon la revendication 1 ou la revendication 2, dans laquelle la nano-suspension présente une stabilité en raison de la dispersibilité uniforme des particules de médicament pendant plus de 20 jours sous une température de 4°C.

6. Procédé de préparation d'une nano-suspension comprenant de la progérine, le procédé comprenant les étapes consistant à :
(i) mélanger de l'eau purifiée avec de l'hydroxypropylméthylcellulose (HPMC) (b) et du succinate de D-α-tocophérol polyéthylène glycol (TPGS) (c) pour préparer une solution de véhicule, puis ajouter et mélanger de la progérine (a) représentée par la formule chimique 1 suivante afin de préparer une suspension ;
(ii) soumettre la suspension à un broyage à billes de type humide pour préparer une nano-suspension ; et
(iii) mélanger la nano-suspension avec du sorbate de potassium (d) pour enfin préparer une nano-suspension avec une stabilité accrue : 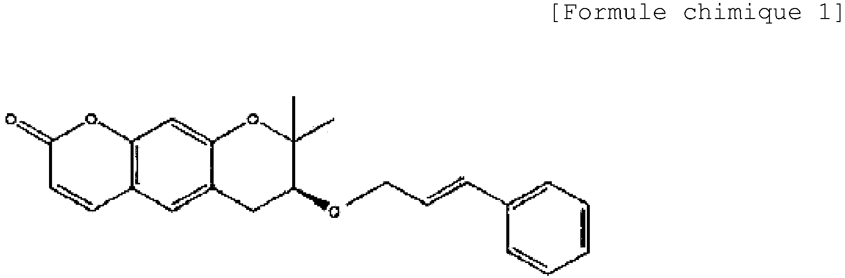 dans laquelle le composé de progérine dans la nano-suspension de (iii) est sous la forme de particules de médicament avec un diamètre de particule moyen (D50) de 100 nm à 300 nm.

7. Procédé de préparation d'une nano-suspension comprenant de la progérine, le procédé comprenant les étapes consistant à :
(i) mélanger de l'eau purifiée avec de l'hydroxypropylcellulose (HPC) (b') ; et du sulfosuccinate de dioctyle et de sodium (DOSS) (c') pour préparer une solution de véhicule, puis ajouter et mélanger de la progérine (a) représentée par la formule chimique 1 suivante afin de préparer une suspension ;
(ii) soumettre la suspension à un broyage à billes de type humide pour préparer une nano-suspension ; et
(iii) mélanger la nano-suspension avec du sorbate de potassium (d) pour enfin préparer une nano-suspension avec une stabilité accrue : 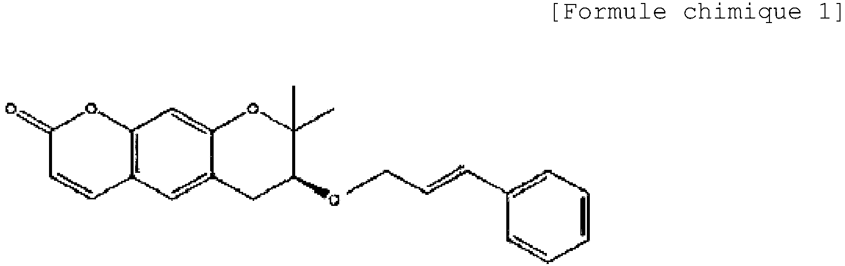 dans laquelle le composé de progérine dans la nano-suspension de (iii) est sous la forme de particules de médicament avec un diamètre de particule moyen (D50) de 100 nm à 300 nm.

8. "Procédé selon la revendication 6 ou la revendication 7, dans lequel la température de l'eau purifiée de (i) est de 50 à 70°C.

9. Procédé selon la revendication 6 ou la revendication 7, dans lequel, lors de l'exécution du broyage à billes de type humide en (ii), la nano-suspension est préparée en mélangeant la suspension de (i) et des billes de zircone à un rapport volumique de 1:1 à 1:5, avant un broyage.

10. Procédé selon la revendication 6 ou la revendication 7, dans lequel la nano-suspension de (iii) comprend des nanoparticules de médicament de progérine présentant un diamètre moyen de particule de 150 nm à 250 nm.
